# EUROPEAN PATENT APPLICATION

(11) **EP 4 087 139 A1**
(43) Date of publication of application: **09.11.2022**
(21) Application number: 19958459.0
(22) Date of filing: 31.12.2019
(51) Int. Cl.: H03M 7/30

(54) **METHOD AND DEVICE FOR CREATING GENE MUTATION DICTIONARY AND USING THE DICTIONARY TO COMPRESS GENOME DATA**

(71) Applicant: MGI Tech Co., Ltd., Shenzhen, Guangdong 518083 (CN)
(72) Inventor: XU, Chongjun, Shenzhen, Guangdong 518083 (CN); ZHOU, Yujun, Shenzhen, Guangdong 518083 (CN); DENG, Ziqing, Shenzhen, Guangdong 518083 (CN); GONG, Meihua, Shenzhen, Guangdong 518083 (CN); JIANG, Hui, Shenzhen, Guangdong 518083 (CN); XU, Xun, Shenzhen, Guangdong 518083 (CN)
(74) Representative: DehnsGermany Partnerschaft von Patentanwälten
(86) International application number: PCT/CN2019/130731
(87) International publication number: WO 2021/134574

(57) **Abstract**

Provided are a method and device for creating a gene mutation dictionary, and a method and device for compressing genomic data using the gene mutation dictionary. The method for creating a gene mutation dictionary includes: obtaining genome sequence data of a plurality of individuals of a species and reference genome data of the species; aligning genome sequence data of each individual to the reference genome data to obtain a mutation result of the genome sequence data of each individual relative to the reference genome data; partitioning a genome of the species into a plurality of unit regions of biological significance; and generating a plurality of mutant patterns of the individuals in each unit region by statistically analyzing mutant status for each unit region based on the mutation result, and numbering the mutant patterns, to obtain the gene mutation dictionary. The present application solves the problems in terms of genomic data compression, reduces the storage size significantly, and greatly reduces the cost of storing data.

## Description

### FIELD

The present disclosure relates to the technical field of genomic data storage, and in particular, to a method and device for creating a gene mutation dictionary, and a method and device for compressing genomic data using the gene mutation dictionary.

### BACKGROUND

The human genome has been mapped. However, the genomes of most individuals have not yet been mapped. Once the individual genome maps are completed, there will be a problem that the existing computer systems around the world would not have enough storage space to store the data. The size of genomic data alone constitutes one of the main obstacles to the true spread of genomics to the world. The raw data of the sequenced human genome may have a size of 200 to 300 gigabytes, while the analyzed genomic data may occupy 1 terabyte. The size of data alone can be a very tricky problem for building a whole genome library.

Storing human genomic data is not just about storage, and the purpose thereof is not only to understand how genes usually interact, but also to be able to apply genomic data to individuals for personalized medical care. For example, the personalized medical care may usher in a thriving era when the genetic maps can be drawn and used by individuals. However, if the existing genomic data are coded to store genetic information, an individual can learn about his or her genetic information by checking his or her own genetic codes, which benefits the general public.

The current method of reducing data storage space is data compression, for example, through data compression tools such as gzip. However, a compression rate of the data compression tools for general programs or texts is only about 60%. The compressed data still occupies a lot of storage space. The data compression is still an utterly inadequate measure when it comes to the dramatic increase in sequencing data.

The data compression may save storage space of genomic data and can hide data in a lossless and transparent manner. However, if an individual wants to view his/her own genetic information, the data must be decompressed. It is troublesome to known an individual's genomic information by repeatedly compressing and decompressing. In addition, decompression still requires large storage to support, and the cost is not particularly reduced.

### SUMMARY

The purpose of the present disclosure is to provide a method for creating a gene mutation dictionary, and a method and device for compressing genomic data using the gene mutation dictionary, which solve the problems in terms of genomic data compression, significantly reduces the storage size, and greatly reduces the cost of data storage.

According to a first aspect of the present disclosure, the present disclosure provides a method for creating a gene mutation dictionary. The method includes: obtaining genome sequence data of a plurality of individuals of a species and reference genome data of the species; aligning genome sequence data of each of the plurality of individuals to the reference genome data to obtain a mutation result of the genome sequence data of each of the plurality of individuals relative to the reference genome data; and partitioning a genome of the species into a plurality of unit regions of biological significance; and generating a plurality mutant patterns of the plurality of individuals in each unit region by statistically analyzing mutant status for each of the plurality of unit regions based on the mutation result, and numbering the mutant patterns, to obtain the gene mutation dictionary. The gene mutation dictionary includes the mutant patterns corresponding to each unit region and index numbers of the mutant patterns.

In a preferred embodiment, the species is the Homo sapiens, and the plurality of individuals includes 1000 or more human individuals.

In a preferred embodiment, the plurality of unit regions includes coding regions, non-coding regions, and genes.

In a preferred embodiment, the number of the plurality of unit regions of biological significance ranges from thousands to tens of thousands.

In a preferred embodiment, the number of the plurality of unit regions of biological significance is 60,000, with a tolerable error range from -10% to 10%.

In a preferred embodiment, the plurality of unit regions includes 30,000 gene coding regions and 30,000 non-coding regions, each with a tolerable error range from -10% to 10%.

In a preferred embodiment, said generating the plurality of mutant patterns by statistically analyzing mutant status for each of the plurality of unit regions, and numbering the mutant patterns includes: for each of the plurality of unit regions, numbering and counting, based on the mutation results of the plurality of individuals, the mutant patterns in accordance with an order of the plurality of individuals, wherein a count is the number of individuals supporting the mutant pattern. If the mutation result of a latter individual is consistent with the mutation result of any previous individual, the mutant pattern and the index number of the previous individual are adopted as those of the latter individual and the count of the mutant pattern is added one. If the mutation result of the latter individual is inconsistent with the mutation result of any previous individual, a new mutant pattern is added to the dictionary, and the new mutant pattern is provided with an index number and counted, until all the mutant patterns for each unit region as well as the index numbers and counts of all the mutant patterns are obtained.

In a preferred embodiment, the above method further includes: sorting the mutant patterns in a descending order of the counts of the mutant patterns, and renumbering the mutant patterns in the sorted order.

According to a second aspect of the present disclosure, the present disclosure provides a device for creating a gene mutation dictionary. The device includes: a data obtaining unit configured to obtain genome sequence data of a plurality of individuals of a species and reference genome data of the species; a data alignment unit configured to align genome sequence data of each of the plurality of individuals to the reference genome data to obtain a mutation result of the genome sequence data of each of the plurality of individuals relative to the reference genome data; a partitioning unit configured to partition a genome of the species into a plurality of unit regions of biological significance; and a dictionary generation unit configured to generate mutant patterns of the plurality of individuals in each unit region by statistically analyzing mutant status for each of the plurality of unit regions based on the mutation result, and number the mutant patterns, to obtain the gene mutation dictionary. The gene mutation dictionary includes the mutant patterns corresponding to each unit region and index numbers of the mutant patterns.

According to a third aspect of the present disclosure, the present disclosure provides a computer-readable storage medium including a program. The program is executable by a processor to implement the method of the first aspect.

According to a fourth aspect of the present disclosure, the present disclosure provides a method for compressing genomic data using a gene mutation dictionary. The method includes: obtaining genome sequencing data of an individual, the genome sequencing data including a plurality of unit regions of biological significance; aligning the genome sequencing data to the gene mutation dictionary generated by the method of the first aspect, to obtain a mutant pattern and an index number of the mutant pattern in each of the plurality of unit regions of the individual, the mutant pattern being consistent with a mutant status of each of the plurality of unit regions; and storing the index number of the mutant pattern for a corresponding one of the plurality of unit regions of the individual instead of storing actually measured mutation.

According to a fifth aspect of the present disclosure, the present disclosure provides a device for compressing genomic data using a gene mutation dictionary. The device includes: a data obtaining unit configured to obtain genome sequencing data of an individual, the genome sequencing data including a plurality of unit regions of biological significance; a data alignment unit configured to align the genome sequencing data to the gene mutation dictionary generated by the method of the first aspect, to obtain a mutant pattern and an index number of the mutant pattern, the mutant pattern being consistent with a mutant status of each of the plurality of unit regions of the individual; and a data storage unit configured to store the index number of the mutant pattern for each of the plurality of unit regions of the individual instead of storing actually measured mutation.

According to a sixth aspect of the present disclosure, the present disclosure provides a computer-readable storage medium including a program. The program is executable by a processor to implement the method of the fourth aspect.

According to a seventh aspect of the present disclosure, the present disclosure provides a method for restoring genomic data compressed using a gene mutation dictionary. The method includes: obtaining compressed genomic data, the compressed genomic data being generated through compression using the gene mutation dictionary generated by the method of the first aspect, the compressed genomic data including index numbers of mutant patterns of a plurality of unit regions in the gene mutation dictionary; finding, from the compressed genomic data, the index number of the mutant pattern for each of the plurality of unit regions in the gene mutation dictionary; and extracting, from the gene mutation dictionary, the mutant pattern corresponding to the index number and a mutation result of the mutant pattern on respective base sites.

According to an eighth aspect of the present disclosure, the present disclosure provides a device for restoring genomic data compressed using a gene mutation dictionary. The device includes: a data obtaining unit configured to obtain compressed genomic data, the compressed genomic data being generated through compression using the gene mutation dictionary generated by the method of the first aspect, the compressed genomic data including index numbers of mutant patterns of a plurality of unit regions in the gene mutation dictionary; an index number obtaining unit configured to find, from the compressed genomic data, the index number of the mutant pattern for each of the plurality of unit regions in the gene mutation dictionary; and a mutation extraction unit configured to extract, from the gene mutation dictionary, the mutant pattern corresponding to the index number and a mutation result of the mutant pattern on respective base sites.

According to a ninth aspect of the present disclosure, the present disclosure provides a computer-readable storage medium including a program. The program is executable by a processor to implement the method of the seventh aspect.

The method of the present disclosure solves the problems in terms of genomic data compression, and it can achieve a compression amount of about 25,000 times, which significantly reduces the storage size and greatly reduces the cost of storing data. With the rapid expansion of sequencing data, the size of genomic data storage has almost reached an incredible level. The genomic data of the whole genome, which is effectively encoded through the method of the present disclosure, can be compressed to a size of about 120kb, which greatly reduces the storage cost of genomic data in addition to the great reduction of the sequencing cost.

### BRIEF DESCRIPTION OF DRAWINGS

FIG. 1 is a flowchart of a method for creating a gene mutation dictionary according to an embodiment of the present disclosure.
FIG. 2 illustrates counts of biotypes of all the human genes according to an embodiment of the present disclosure.
FIG. 3 is a structural block diagram of a device for creating a gene mutation dictionary according to an embodiment of the present disclosure.
FIG. 4 is a flowchart of a method for compressing genomic data using a gene mutation dictionary according to an embodiment of the present disclosure.
FIG. 5 is a structural block diagram of a device for compressing genomic data using a gene mutation dictionary according to an embodiment of the present disclosure.
FIG. 6 is a flowchart of a method for restoring genomic data compressed using a gene mutation dictionary according to an embodiment of the present disclosure.
FIG. 7 is a structural block diagram of a device for restoring genomic data compressed using a gene mutation dictionary according to an embodiment of the present disclosure.
FIG. 8 illustrates a list of mutant patterns of Chr21_KRTAP12-4 gene in 1,000 Genomes Project according to an embodiment of the present disclosure.
FIG. 9 is a schematic diagram of a typical gene mutation dictionary according to an embodiment of the present disclosure.
FIG. 10 is a schematic diagram of a storage process of a human gene mutation dictionary according to an embodiment of the present disclosure.

### DESCRIPTION OF EMBODIMENTS

The present disclosure is described in detail below by means of specific embodiments with reference to the accompanying drawings. In the following embodiments, many details are described to facilitate the understanding of the present disclosure. However, those skilled in the art can readily recognize that some of these features may be omitted under different circumstances, or may be replaced by other materials and methods.

In addition, the features, operations, or characteristics described in the specification may be combined in any suitable manner to constitute various embodiments. At the same time, the steps or actions in the description involving method may also be exchanged or adjusted in order in a manner apparent to those skilled in the art. Therefore, the various sequences in the specification and drawings are only for the purpose of clearly describing a certain embodiment and are not meant to be a necessary order, unless it is specified that a certain sequence must be followed.

In the era with a continuous increase of individual genomic data, many companies and institutions give priority to solving the problem of data compression by means of computers and high-performance storage. Since merely the data format has been changed without changing the data, the storage size is not significantly reduced.

The method of the present disclosure, different from the common data compression for reducing the storage space, uses bioinformatics to compress the sequence information (about 3GB) of the human genome from nearly 3,000,000,000 bases to about only 120KB, which is nearly 25,000 times of data compression without loss of genetic information.

In order to compress genomic data, the method of the present disclosure mainly includes three technical solutions.
(1) Multiple biologically significant patterns are created and encoded with numbers to generate a gene mutation dictionary. In some embodiments, the whole human genome has about 30,000 genes. After the 1,000 human genomes, 10,000 human genomes, and even 1 million human genomes are sequenced, a mutation dictionary is created based on all the determined mutants for each gene as a unit. In some embodiments, for non-coding regions such as introns, each 100kb sequence can serve as a unit (3GB human genome will have 30,000 units of 100kb), and a mutant dictionary is created with mutants after sequencing, each gene as a unit. In this way, the whole human genome can be divided into 60,000 units, including 30,000 coding gene regions and 30,000 non-coding regions. In other embodiments, the number of units may be appropriately increased or reduced according to the mutation patterns of the genome. According to the mutation patterns in each unit, 2⁸ (=256) mutation patterns take 1 byte for storage; 2¹⁶ (=65,536) mutation patterns take 2 bytes for storage; and 2²⁴ (= 16,777,216) mutation patterns take 3 bytes for storage; the gene mutation dictionary may be stored in a large database.
(2) After the individual genome is sequenced, only the reference numbers of patterns of each unit are stored according to the gene mutation dictionary, instead of storing the actual detected mutations. If each pattern is represented by 2 bytes, each person's whole genome only needs a storage size of 60000^{∗}2 = 120kb, thereby achieving nearly 25,000 times of data compression.
(3) Gene mutation information is obtained. Based on the stored reference numbers of gene patterns and the gene mutation dictionary, the results of gene sequencing can also be completely restored.

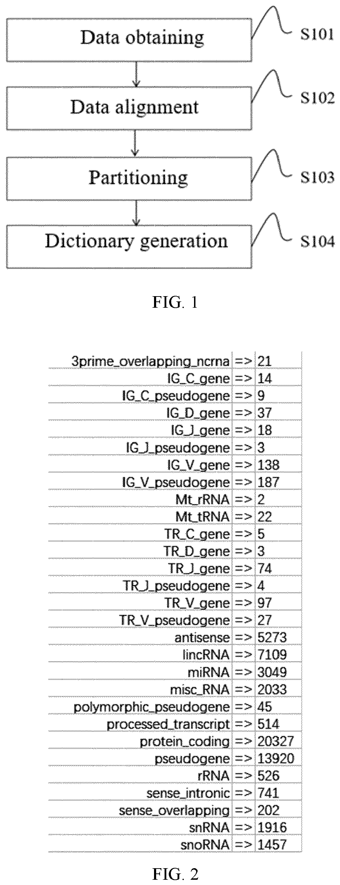

As illustrated in FIG. 1, an embodiment of the present disclosure provides a method for creating a gene mutation dictionary, including the following steps:

### S101: Data obtaining

Genome sequence data of a plurality of individuals of a species and reference genome data of the species are obtained.

The method for creating a gene mutation dictionary according to the present disclosure is applicable to any species, especially the species having the storage requirements of individual genome sequencing data, mainly the storage of human genomic data. The method of the present disclosure requires genome sequence data of multiple individuals, for example, genome sequence data of more than 1,000 people. Generally speaking, the more individual samples, the more comprehensive the mutant patterns are in the created gene mutation dictionary. Generally speaking, in some embodiments, the genome data information of at least 1,000 people should be included, and the genome data information of more than one million people is optimal. Accordingly, the reference genome data is human reference genome data.

### S102: Data alignment

The genome sequence data of each of the plurality of individuals is aligned to the reference genome data to obtain a mutation result of the genome sequence data of each of the plurality of individuals relative to the reference genome data.

In the embodiments of the present disclosure, the genome sequence alignment can be performed with various methods, for example, by means of sequence alignment software such as BWA or SOAP2. The mutation result of each individual's genome sequence data relative to the reference genome data is obtained through the sequence alignment, and the mutation result is reflected by a difference between the base of the individual's genome and the base of the reference genome at each base site.

### S103: Partitioning

The genome of said species is partitioned into a plurality of unit regions of biological significance.

In the embodiments of the present disclosure, the biologically significant unit regions may include coding regions, non-coding regions, genes, etc. on the genome. In other embodiments, the biologically significant unit regions may be genes of all the human biotypes, etc.

In the embodiments of the present disclosure, the number of the plurality of unit regions of biological significance may be thousands to tens of thousands. For example, in some embodiments, the number of the plurality of unit regions is 60,000, with a tolerable error range from -10% to 10%, for example, 54,000 to 66,000 biologically significant unit regions. In a preferred embodiment of the present disclosure, the biologically significant unit regions include 30,000 gene coding regions and 30,000 non-coding regions, each with a tolerable error range from -10% to 10%.

For example, in an embodiment, based on the biologically significant unit regions such as coding regions, non-coding regions, genes, etc., the entire human genomic data (about 3GB) is partitioned into about 60,000 unit regions, among which 30,000 unit regions are the gene coding regions and the other 30,000 unit regions are the non-coding regions. Each non-coding region has a size of about 100kb.

### S104: Dictionary generation

Mutant patterns of the plurality of individuals in each unit region are generated by statistically analyzing mutant status for each of the plurality of unit regions based on the mutation result, and the mutant patterns are numbered, to obtain the above-mentioned gene mutation dictionary. The gene mutation dictionary includes the mutant patterns corresponding to each unit region and index numbers of the mutant patterns.

In the embodiments of the present disclosure, a mutant pattern refers to a specific combination of mutation sites in a specific unit region of biological significance, including positions and base changes of the mutation sites in the unit region. There is at least one mutation site difference between any two mutant patterns. That is, as long as at least one mutation site difference is present, these two mutant patterns are different mutant patterns.

In the embodiments of the present disclosure, the numbering of the mutant patterns means that the mutant types are sequentially coded in order. A typical but non-limiting coding manner is to use a combination of an index number of a biologically significant unit region and an index number of a mutant pattern corresponding thereto as a code of a particular mutant pattern.

In an embodiment of the present disclosure, said generating the plurality of mutant patterns by statistically analyzing mutant status for each unit region, and numbering the mutant patterns include the following step: for each of the unit regions, numbering and counting, based on the mutation results of the plurality of individuals, the mutant patterns in accordance with an order of the plurality of individuals. A count is the number of individuals supporting the mutant pattern. If the mutation result of a latter individual is consistent with the mutation result of any previous individual, the mutant pattern and the index number of the previous individual are adopted as those of the latter individual and the count of the mutant pattern is added one. If the mutation result of the latter individual is inconsistent with the mutation result of any previous individual, a new mutant pattern is provided with an index number and counted, until all the mutant patterns for each unit region as well as the index numbers and counts of all the mutant patterns are obtained. Further, the above method further includes: sorting the mutant patterns in a descending order of the counts of the mutant patterns, and renumbering the mutant patterns in the sorted order.

For example, in an embodiment of the present disclosure, based on the mutation result analyzed in the previous step, the mutant status for each of about 60,000 unit regions of the human genome is statistically analyzed. Each unit region (gene or intron) is statistically analyzed as follows. For a person-1, the mutation result (a mutation combination of all mutation sites) obtained through the alignment is denoted as pattern-1, the count of pattern-1 is recorded as 1; for a next person-2, this unit region is aligned, and if the mutation result of the person-2 obtained through the alignment is consistent with the mutation result of the person-1, the same pattern, i.e., the pattern-1, is obtained, and thus the count of pattern-1 is 1+1=2, and so on; or if this unit region of the person-2 is aligned and a mutation result inconsistent with that of the person-1 is obtained, the mutation result is recorded as a new pattern, i.e., pattern-2, and a count of pattern-2 is recorded as 1, until the individual samples in the database are all statistically analyzed. In order to accelerate the retrieval of the subsequent gene mutation dictionary, in the preferred method of the present disclosure, the patterns are sorted based on the counts thereof in a descending order, and the pattern with the highest count is redefined as pattern-1, the pattern with a second highest count is defined as pattern-2, and so on.

In the embodiments of the present disclosure, the biologically significant unit regions may be partitioned according to other biological criteria, such as the counts of biotypes of human genes. For example, FIG. 2 illustrates the counts of biotypes of human genes.

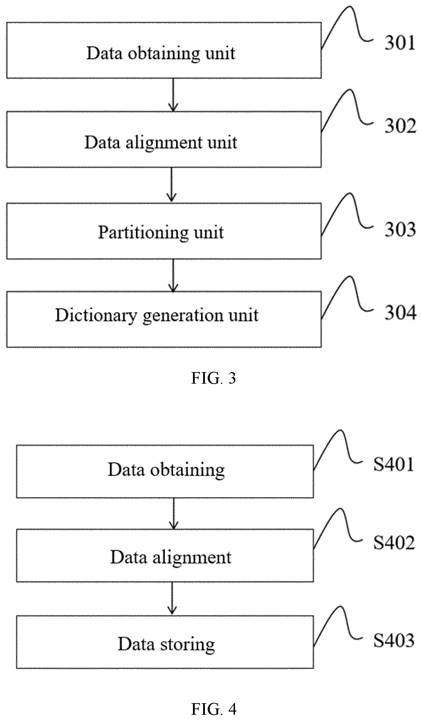

The present disclosure further provides a device for creating a gene mutation dictionary, corresponding to the method for creating the gene mutation dictionary of the present disclosure. As illustrated in FIG. 3, the device includes: a data obtaining unit 301 configured to obtain genome sequence data of a plurality of individuals of a species and reference genome data of the species; a data alignment unit 302 configured to align genome sequence data of each of the plurality of individuals to the reference genome data to obtain a mutation result of the genome sequence data of each of the plurality of individuals relative to the reference genome data; a partitioning unit 303 configured to partition a genome of the species into a plurality of unit regions of biological significance; and a dictionary generation unit 304 configured to generate mutant patterns of the plurality of individuals in each unit region by statistically analyzing mutant status for each of the plurality of unit regions based on the mutation result, and number the mutant patterns, to obtain the gene mutation dictionary, the gene mutation dictionary including the mutant patterns corresponding to each unit region and index numbers of the mutant patterns.

Those skilled in the art can understand that all or part of the functions of the various methods in the foregoing embodiments may be implemented by means of hardware or by means of computer programs. When all or part of the functions in the above embodiments are implemented by means of a computer program, the program may be stored in a computer-readable storage medium. The storage medium may include read-only memory, random access memory, magnetic disk, optical disk, hard disk, etc. The program is executed by a computer to exert the above-mentioned functions. For example, the program is stored in a memory of the device, and when the program in the memory is executed by a processor, all or part of the above functions can be realized. In addition, when all or part of the functions in the above-mentioned embodiments are implemented by means of a computer program, the program may be stored in a server, another computer, or a storage medium such as a magnetic disk, an optical disk, a flash disk or a mobile hard disk, and the program can be downloaded or copied to be saved in a memory of a local device or it can update the version of the system of the local device. The program in the memory, when being executed by a processor, can implement all or part of the functions in the above embodiments.

Therefore, an embodiment of the present disclosure provides a computer-readable storage medium including a program, which is executable by a processor to implement the method for creating the gene mutation dictionary of the present disclosure.

On the basis of the gene mutation dictionary of the present disclosure, the present disclosure further provides a method for compressing genomic data using the gene mutation dictionary. As illustrated in FIG. 4, the method includes the following steps.

### S401: Data obtaining

Genome sequencing data of an individual is obtained. The genome sequencing data includes a plurality of unit regions of biological significance.

### S402: Data alignment

The genome sequencing data is aligned to the gene mutation dictionary generated by the method for creating the gene mutation dictionary according to the present disclosure, to obtain a mutant pattern and an index number of the mutant pattern, which is consistent with a mutant status of each of the plurality of unit regions of the individual.

### S403: Data storing

The index number of the mutant pattern for each unit region of the individual is stored instead of storing actually measured mutation.

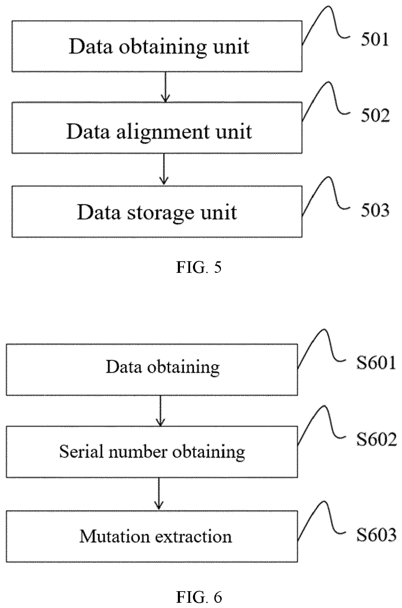

The present disclosure further provides a device for compressing genomic data using a gene mutation dictionary. As illustrated in FIG. 5, the device includes: a data obtaining unit 501 configured to obtain genome sequencing data of an individual, the genome sequencing data including a plurality of unit regions of biological significance; a data alignment unit 502 configured to align the genome sequencing data to the gene mutation dictionary generated by the method for creating the gene mutation dictionary according to the present disclosure, to obtain a mutant pattern and an index number of the mutant pattern, which is consistent with a mutant status of each of the plurality of unit regions of the individual; and a data storage unit 503 configured to store the index number of the mutant pattern for each of the plurality of unit regions of the individual instead of storing actually measured mutation.

The present disclosure further provides a computer-readable storage medium including a program, which is executable by a processor to implement the method for compressing genomic data using the gene mutation dictionary according to the present disclosure.

The present disclosure further provides a method for restoring genomic data compressed by using the gene mutation dictionary. As illustrated in FIG. 6, the method includes the following steps.

### S601: Data obtaining

Compressed genomic data, which is generated through compression using the gene mutation dictionary generated by the method for creating the gene mutation dictionary according to the present disclosure, is obtained. The compressed genomic data includes index numbers of mutant patterns of a plurality of unit regions in the gene mutation dictionary.

### S602: Index number obtaining

The index number of the mutant pattern for each of the plurality of unit regions in the gene mutation dictionary is found from the compressed genomic data.

### S603: Mutation extraction

The mutant pattern corresponding to the index number and a mutation result of the mutant pattern on respective base sites are extracted from the gene mutation dictionary.

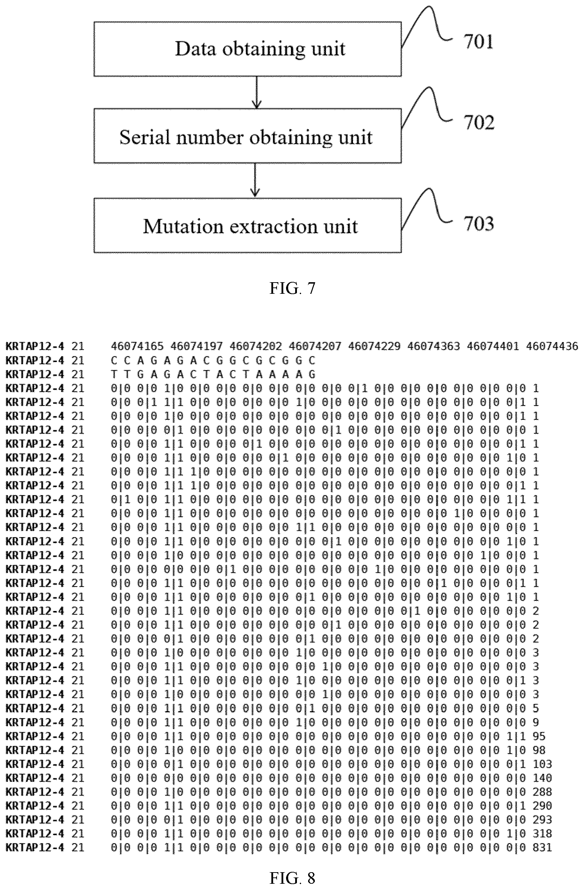

The present disclosure further provides a device for restoring genomic data compressed by using a gene mutation dictionary. As illustrated in FIG. 7, the device includes: a data obtaining unit 701 configured to obtain compressed genomic data, which is generated through compression using the gene mutation dictionary generated by the method for creating the gene mutation dictionary according to the present disclosure, and which includes index numbers of mutant patterns of a plurality of unit regions in the gene mutation dictionary; an index number obtaining unit 702 configured to find, from the compressed genomic data, the index number of the mutant pattern for each of the plurality of unit regions in the gene mutation dictionary; and a mutation extraction unit 703 configured to extract, from the gene mutation dictionary, the mutant pattern corresponding to the index number and a mutation result of the mutant pattern on respective base sites.

According to a ninth aspect of the present disclosure, the present disclosure provides a computer-readable storage medium including a program, which is executable by a processor to implement the method for restoring genomic data compressed by using the gene mutation dictionary according to the present disclosure.

The technical solutions and effects of the present disclosure will be described in detail below by means of examples. It should be understood that these examples are merely illustrative and should not be construed as limiting the present disclosure.

### Example 1: Creation of Gene Mutation Dictionary

(1) Gene database was collected. In the example of the present disclosure, a gene database of 1,000 Genomes Project was collected, and gene data was collected.
(2) The collected genetic data was aligned to the human reference genome.
(3) The collected whole human genomic data was partitioned into approximately 60,000 biologically significant unit regions such as coding regions, non-coding regions, genes, etc., among which 30,000 unit regions were gene coding regions and the other 30,000 unit regions were non-coding regions. Each non-coding region had a size of about 100 kb.
(4) Based the mutation result analyzed in the previous step, the mutant status for each of about 60,000 unit regions of the human genome was statistically analyzed. Each unit region (gene or intron) was statistically analyzed as follows. For a person-1, the mutation result (a mutation combination of all mutation sites) obtained through the alignment was denoted as pattern-1, the count of pattern-1 was recorded as 1; for a next person-2, this unit region was aligned, and if the mutation result of the person-2 obtained through the alignment was consistent with the mutation result of the person-1, the same pattern, i.e., the pattern-1, was obtained, and thus the count of pattern-1 is 1+1=2, and so on; or if this unit region of the person-2 was aligned and a mutation result inconsistent with that of the person-1 was obtained, the mutation result was recorded as a new pattern, i.e., pattern-2, and the count of pattern-2 was recorded as 1, until the individual samples in the database were all statistically analyzed. In order to accelerate the retrieval of the subsequent gene mutation dictionary, in the preferred method of the present disclosure, the patterns are sorted based on the counts thereof in a descending order, and the pattern with the highest count is redefined as pattern-1, the pattern with a second highest count is defined as pattern-2, and so on.

In the present example, mutations of all the coding regions (about 30,000 genes) of the genome information of 2,504 people (data from the library of 1,000 Genomes Project) were analyzed. Mutation analysis was performed on each gene of these 2,504 people. According to the numbering method in step (4) of the present example, each gene can finally obtain a maximum of 2,504 mutant patterns (when each individual has a different mutation).

As an example, for the KRTAP12-4 gene, a mutant dictionary was created and mutants were statistically analyzed based on VCF files of 2,504 samples of the 1,000 Genomes Project. The results of statistical analysis indicated that, among the 2,504 samples, 831 samples had the same mutant pattern of the KRTAP12-4 gene with a highest mutation frequency, that is, a homozygous mutation from base A to base G occurred at position 460742022 of chromosome NO. 21; 318 samples (12.7%) had a mutation with the second highest mutation frequency; and 16 samples has unique patterns. It is worth noting that the 2504 samples had only 33 mutant patterns, and the data size thereof was further compressed by 75 times when compared to the VCF files, which were already compressed to a great extent. FIG. 8 illustrates a schematic diagram of a result of the mutant pattern list of the Chr21_KRTAP12-4 gene in the 1,000 Genomes Project according to an embodiment of the present disclosure.

Table 1 lists the statistically analysis results of mutant patterns of 30 genes that are randomly selected from nearly 30,000 genes in 2,504 human samples of the database of 1,000 Genomes Project. It is clear that the number of the mutant patterns of the mutants is significantly smaller than the number of the samples. It indicates that for a certain mutant pattern, multiple people have exactly the same mutation result. When the mutant pattern is stored instead of storing respective sample mutants, the required storage space can be greatly reduced.

**[Table 1] Statistically analysis results of mutant patterns of 30 genes randomly selected from the database of the 1,000 Genomes Project**

| Gene ID | Chromosome | Count of SNP pattern | Gene length |
|---|---|---|---|
| AL645608.2 | 1 | 104 | 1940 |
| PRAMEF14 | 1 | 64 | 5242 |
| PRAMEF19 | 1 | 67 | 3516 |
| C2CD4D | 1 | 181 | 2738 |
| MGC10955 | 2 | 104 | 922 |
| IQCF5 | 3 | 107 | 1863 |
| HSD17B8 | 6 | 181 | 2189 |
| RP11-521M14.2 | 8 | 61 | 1072 |
| DKFZP667F0711 | 10 | 180 | 2445 |
| CHCHD1 | 10 | 63 | 1605 |
| HUG1 | 10 | 62 | 1088 |
| AL355531.2 | 10 | 107 | 641 |
| OR4C16 | 11 | 182 | 932 |
| OR5D13 | 11 | 68 | 944 |
| C11orf31 | 11 | 182 | 2161 |
| OR5B12 | 11 | 65 | 1053 |
| GNG3 | 11 | 66 | 1543 |
| PPP1R14B | 11 | 105 | 2457 |
| CARD18 | 11 | 182 | 2005 |
| OR6C70 | 12 | 104 | 938 |
| RPS26 | 12 | 183 | 2479 |
| RHOV | 15 | 105 | 2075 |
| MT1H | 16 | 179 | 1315 |
| KRTAP9-3 | 17 | 106 | 990 |
| NT5C | 17 | 182 | 1570 |
| RP4-583P15.14 | 20 | 68 | 2128 |
| AC016586.1 | 19 | 183 | 2027 |
| ADM5 | 19 | 106 | 1911 |
| RIMBP3B | 22 | 68 | 5783 |
| KRTAP10-8 | 21 | 105 | 875 |

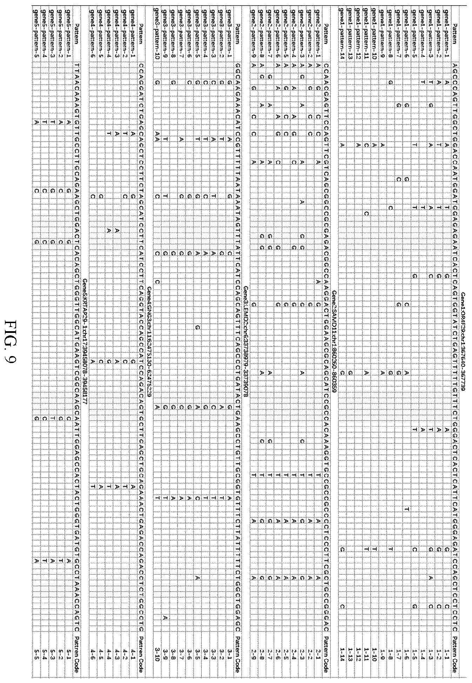

In the present example, the human genomic data in the database was aligned, and the mutant patterns were classified for each unit region. The genes of all the people were numerated in the above manner and collected to obtain a complete gene mutation dictionary. A typical gene mutation dictionary is similar to the form in FIG. 9.

In the present example, for instance, each unit region was stored with 2 bytes, which can store 65,536 mutant patterns, and thus can store biologically significant mutants. If a unit region had fewer than 65,536 mutant patterns, the remaining mutant patterns can be used for new patterns discovered later. If a certain unit had more than 65,536 mutant patterns, the mutant patterns in this unit can be stored with 3 bytes, and in this case, 16,777,216 mutant patterns are included.

### Example 2: Storage of Human Individual Genomic data

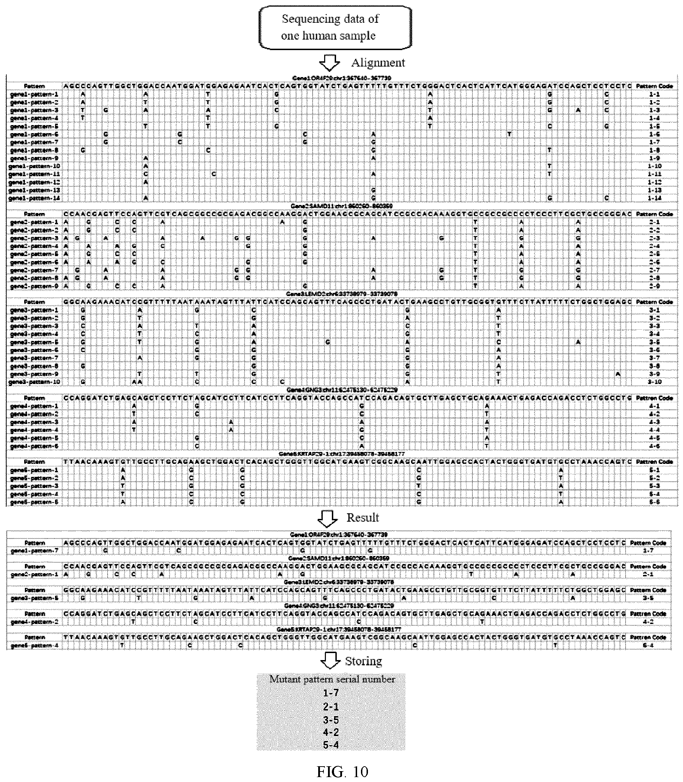

After the human genome of an individual was sequenced, the sequencing result was aligned to the gene mutation dictionary for each unit region, as illustrated in FIG. 10. In the gene mutation dictionary, instead of the actually measured mutations of the unit region, only the index numbers of mutant patterns of each unit region were stored.

FIG. 10 illustrates a storage process of a human gene mutation dictionary. For example, for the first 100 bp mutant patterns of genes OR4F29, SAMD11, LEMD2, GNG3, KRTAP29-1, these five genes of one person were analyzed. For gene 1, it was determined through alignment that pattern-7 was completely consistent; for gene 2, it was determined through alignment that pattern-2 was completely consistent; for gene 3, it was determined through alignment that pattern-5 was completely consistent; for gene 4, it was determined through alignment that pattern-2 was completely consistent; and for gene 5, it was determined through alignment that pattern-4 was completely consistent. Then, the mutation information of the 5 genes of this individual can be stored as index numbers for the 5 mutant patterns, and the mutation information of each gene of this individual was stored as a corresponding mutant pattern index number, which can save a lot of storage.

In the present example, a typical human mutant sequence storage format is as follows:
1.7
2.1
3.5
4.2
5.4
...
60000.35

The number before the decimal point is an index of the unit region, and the number after the decimal point is an index of the corresponding mutant pattern.

If the mutant pattern of each gene is represented by 2 bytes, the whole genome of each person is divided into 60,000 genes, which requires a storage size of only 60,000^{∗}2 = 120kb, achieving nearly 25,000 times of data compression.

### Example 3: Restoration of Gene Mutant Information

In the present example, the gene sequencing result can also be completely restored based on the stored index numbers of the gene mutant patterns and the gene mutation dictionary.

Since each unit region (e.g., gene) in the gene mutation dictionary corresponds to a fixed region in the whole genome, the mutants corresponding to the mutant patterns of each unit region are fixed. Therefore, based on the index of the unit region for gene storage and the index of the corresponding mutant patterns, the actual mutations can be deduced directly.

### Example 4: Use in Precision Medicine

The whole human genome sequencing will become one of the human physical examination items. The storage of human mutants based on the gene mutation dictionary would be similar to the data format of FIG. 9. When a person is born, he or she can have a high-depth genome sequencing to establish his/her genetic sequence reference. Thereafter, a physical examination of the whole genome sequencing can be carried out every year or at an appropriate time. Most of the time, there may be no gene mutation. Once a mutation occurs, a mutant pattern thereof may be significantly different from that at birth, so as to easily identify the gene mutation.

As illustrated in Table 2, an example of storage of physical examination items for one individual's whole human genome sequencing is illustrated, in which the first column lists the indexes of gene units, and the second and following columns list the number of indexes of mutant patterns corresponding to the respective indexes of the gene units.

**Table 2 Storage examples of human whole-genome sequencing mutants for precision medicine**

| Index of gene unit | At birth | 1 year old | 2 years old | ... | 40 years old | 41 years old |
|---|---|---|---|---|---|---|
| 1 | 20 | 20 | 20 | ... | 20 | 20 |
| 2 | 30 | 30 | 30 | ... | 30 | 30 |
| 2 | 32 | 32 | 32 | ... | 32 | 32 |
| 3 | 40 | 40 | 40 | ... | 40 | 40 |
| 4 | 22 | 22 | 22 | ... | 22 | 22 |
| 5 | 45 | 45 | 45 | ... | 49 | 49 |
| 6 | 123 | 123 | 123 | ... | 123 | 123 |
| 7 | 67 | 67 | 67 | ... | 67 | 67 |
| 8 | 2345 | 2345 | 2345 | ... | 2345 | 2345 |
| 9 | 3214 | 3214 | 3214 | ... | 3214 | 3214 |
| 10 | 112 | 112 | 112 | ... | 112 | 112 |
| 11 | 22 | 22 | 22 | ... | 22 | 22 |
| 12 | 34 | 34 | 34 | ... | 34 | 34 |
| 13 | 56 | 56 | 56 | ... | 56 | 56 |
| 14 | 789 | 789 | 789 | ... | 789 | 789 |
| 15 | 991 | 991 | 991 | ... | 991 | 23 |
| 16 | 123 | 123 | 123 | ... | 123 | 123 |
| 17 | 135 | 135 | 135 | ... | 135 | 135 |
| ... | ... | ... | ... | ... | ... | ... |
| 60000 | 123 | 123 | 123 | ... | 123 | 123 |

The above specific examples are intended to illustrate the present disclosure and merely to help understand the present disclosure, rather than limiting the present disclosure. For those skilled in the art to which the present disclosure pertains, several simple deductions, modifications or substitutions can also be made based on the concept of the present disclosure.

## Claims

1. A method for creating a gene mutation dictionary, comprising:
obtaining genome sequence data of a plurality of individuals of a species and reference genome data of the species;
aligning genome sequence data of each of the plurality of individuals to the reference genome data to obtain a mutation result of the genome sequence data of each of the plurality of individuals relative to the reference genome data; and
partitioning a genome of the species into a plurality of unit regions of biological significance; and
generating a plurality of mutant patterns of the plurality of individuals in each unit region by statistically analyzing mutant status for each of the plurality of unit regions based on the mutation result, and numbering the mutant patterns, to obtain the gene mutation dictionary, wherein the gene mutation dictionary comprises the mutant patterns corresponding to each unit region and index numbers of the mutant patterns.

2. The method according to claim 1, wherein the species is the Homo sapiens, and the plurality of individuals comprises 1,000 or more human individuals.

3. The method according to claim 1, wherein the plurality of unit regions comprises coding regions, non-coding regions, and genes.

4. The method according to claim 1, wherein the number of the plurality of unit regions of biological significance ranges from thousands to tens of thousands.

5. The method according to claim 4, wherein the number of the plurality of unit regions of biological significance is 60,000, with a tolerable error range from -10% to 10%.

6. The method according to claim 5, wherein the plurality of unit regions comprises 30,000 gene coding regions and 30,000 non-coding regions, each with a tolerable error range from - 10% to 10%.

7. The method according to claim 1, wherein said generating the plurality of mutant patterns by statistically analyzing mutant status for each of the plurality of unit regions, and numbering the mutant patterns comprises:
for each of the plurality of unit regions, numbering and counting, based on the mutation results of the plurality of individuals, the mutant patterns in accordance with an order of the plurality of individuals, wherein a count is the number of individuals supporting the mutant pattern, and wherein if the mutation result of a latter individual is consistent with the mutation result of any previous individual, the mutant pattern and the index number of the previous individual are adopted as those of the latter individual and the count of the mutant pattern is added one; if the mutation result of the latter individual is inconsistent with the mutation result of any previous individual, a new mutant pattern is added to the dictionary, and the new mutant pattern is provided with an index number and counted, until all the mutant patterns for each unit region as well as the index numbers and counts of all the mutant patterns are obtained.

8. The method according to claim 7, further comprising:
sorting the mutant patterns in a descending order of the counts of the mutant patterns, and renumbering the mutant patterns in the sorted order.

9. A device for creating a gene mutation dictionary, the device comprising:
a data obtaining unit configured to obtain genome sequence data of a plurality of individuals of a species and reference genome data of the species;
a data alignment unit configured to align genome sequence data of each of the plurality of individuals to the reference genome data to obtain a mutation result of the genome sequence data of each of the plurality of individuals relative to the reference genome data;
a partitioning unit configured to partition a genome of the species into a plurality of unit regions of biological significance; and
a dictionary generation unit configured to generate mutant patterns of the plurality of individuals in each unit region by statistically analyzing mutant status for each of the plurality of unit regions based on the mutation result, and number the mutant patterns, to obtain the gene mutation dictionary, wherein the gene mutation dictionary comprises the mutant patterns corresponding to each unit region and index numbers of the mutant patterns.

10. A computer-readable storage medium, comprising a program, wherein the program is executable by a processor to implement the method according to any one of claims 1 to 8.

11. A method for compressing genomic data using a gene mutation dictionary, the method comprising:
obtaining genome sequencing data of an individual, the genome sequencing data comprising a plurality of unit regions of biological significance;
aligning the genome sequencing data to the gene mutation dictionary generated by the method according to any one of claims 1 to 8, to obtain a mutant pattern and an index number of the mutant pattern in each of the plurality of unit regions of the individual, the mutant pattern being consistent with a mutant status of each of the plurality of unit regions; and
storing the index number of the mutant pattern for each of the plurality of unit regions of the individual instead of storing actually measured mutation.

12. A device for compressing genomic data using a gene mutation dictionary, the device comprising:
a data obtaining unit configured to obtain genome sequencing data of an individual, the genome sequencing data comprising a plurality of unit regions of biological significance;
a data alignment unit configured to align the genome sequencing data to the gene mutation dictionary generated by the method according to any one of claims 1 to 8, to obtain a mutant pattern and an index number of the mutant pattern, the mutant pattern being consistent with a mutant status of a corresponding one of the plurality of unit regions of the individual; and
a data storage unit configured to store the index number of the mutant pattern for each of the plurality of unit regions of the individual instead of storing actually measured mutation.

13. A computer-readable storage medium, comprising a program, wherein the program is executable by a processor to implement the method according to claim 11.

14. A method for restoring genomic data compressed using a gene mutation dictionary, the method comprising:
obtaining compressed genomic data, the compressed genomic data being generated through compression using the gene mutation dictionary generated by the method according to any one of claims 1 to 8, the compressed genomic data comprising index numbers of mutant patterns of a plurality of unit regions in the gene mutation dictionary;
finding, from the compressed genomic data, the index number of the mutant pattern for each of the plurality of unit regions in the gene mutation dictionary; and
extracting, from the gene mutation dictionary, the mutant pattern corresponding to the index number and a mutation result of the mutant pattern on respective base sites.

15. A device for restoring genomic data compressed using a gene mutation dictionary, the device comprising:
a data obtaining unit configured to obtain compressed genomic data, the compressed genomic data being generated through compression using the gene mutation dictionary generated by the method according to any one of claims 1 to 8, the compressed genomic data comprising index numbers of mutant patterns of a plurality of unit regions in the gene mutation dictionary;
an index number obtaining unit configured to find, from the compressed genomic data, the index number of the mutant pattern for each of the plurality of unit regions in the gene mutation dictionary; and
a mutation extraction unit configured to extract, from the gene mutation dictionary, the mutant pattern corresponding to the index number and a mutation result of the mutant pattern on respective base sites.

16. A computer-readable storage medium, comprising a program, wherein the program is executable by a processor to implement the method according to claim 14.
